# EUROPEAN PATENT APPLICATION

(11) **EP 2 711 226 A1**
(43) Date of publication of application: **26.03.2014**
(21) Application number: 13184703.0
(22) Date of filing: 17.09.2013
(51) Int. Cl.: B60K 28/06, F16D 48/06, B60W 40/08, A61B 5/18

(54) **Method, computer program, control device, system and vehicle with such a system for measuring a physiologic property of a driver and for adaptating control of a clutch**

(30) Priority: 24.09.2012 SE 1251068
(71) Applicant: Scania CV AB, 151 87 Södertälje (SE)
(72) Inventor: Eskilson, Anders, 120 48 Enskede gård (SE)

(57) **Abstract**

Method (400), control device (240) and measuring device (230) for the adjustment of a control algorithm comprising at least one driver dependent parameter, which control algorithm controls a clutch control system (250) in a vehicle (220). The method comprises measurement (401) of at least one physical property of the driver (210), establishment (402) of the driver's stress level, based on the measurement carried out (401) and adjustment (403) of the control algorithm to the driver's established (402) stress level.

## Description

### TECHNICAL FIELD

The invention's objective is a method, a measuring device and a control device in a vehicle. Specifically, the invention provides a mechanism for the adjustment of a control algorithm which controls a clutch control system in a vehicle to a stress level in the vehicle's driver.

### BACKGROUND

For all control systems in a vehicle with which the vehicle's driver interacts, it is fundamental to attempt to carry out the driver's commands in the manner believed to be desired by the driver. Vehicle in this context means for example a truck, articulated truck, light goods vehicle, commercial vehicle, minivan, camper, pick-up, work truck, passenger care, emergency vehicle, vehicle, van, ATV, skidder, excavator, car, crane truck, tanker, motorcycle, wheel loader, moped, scooter, limousine, sports car, racer car, radio car, lawnmower, combat vehicle, snowmobile, snow cat, cross-country vehicle, caterpillar, tractor, go-cart, bus, combine harvester, farm machinery or similar motorised manned or unmanned vehicle, designed for land-based geographic transportation.

In a vehicle of the type mentioned above, a number of different drive line configurations may be used. For example, the gearbox may consist of a manual speed gearbox, an automated gearbox or an automatic transmission. With respect to heavy goods vehicles, it is often desirable that these should be able to be driven as comfortably as possible for the driver, which usually means that the gearbox speed changes are carried out automatically with the help of the vehicle's control system. Automated gearboxes have thus become increasingly common in this type of vehicle.

Such automated gear transmission in heavy goods vehicles often consists of control system controlled gear transmission of "manual" " gearboxes (also called AMT, Automated Manual Transmission), e. g. because these are significantly cheaper to manufacture, but also because of their higher efficiency, compared to traditional automatic transmission. They also weigh less. As regards heavy goods vehicles, which are largely used on country roads/motorways, automated manual transmission is therefore usually used. These are usually called automated gearboxes.

The gear transmission change may occur in various ways, and according to one such way, an automated clutch transmission of the vehicle's control system is used when to change gears, so that the driver only needs access to the accelerator and brake pedals.

In principle, the clutch need only be used to start the vehicle from still-standing, since other gear transmission changes may be carried out by the vehicle's control system without the clutch transmission being used, as the gear transmission changes are carried out "without torque". It is also possible for the automated clutch only to be used only for certain gear steps, or only for certain gear changes.

Many times, however, the automated clutch is used for all or substantially all gear changes, for comfort reasons.

The automated clutch is controlled by controlling a clutch actuator with the help of the vehicle's control system. The clutch actuator may for example consist of one or several pneumatically controlled pistons acting on a lever, so that the clutch is opened/closed by the said pistons effectuating a lever movement. The clutch actuator may also be electrical.

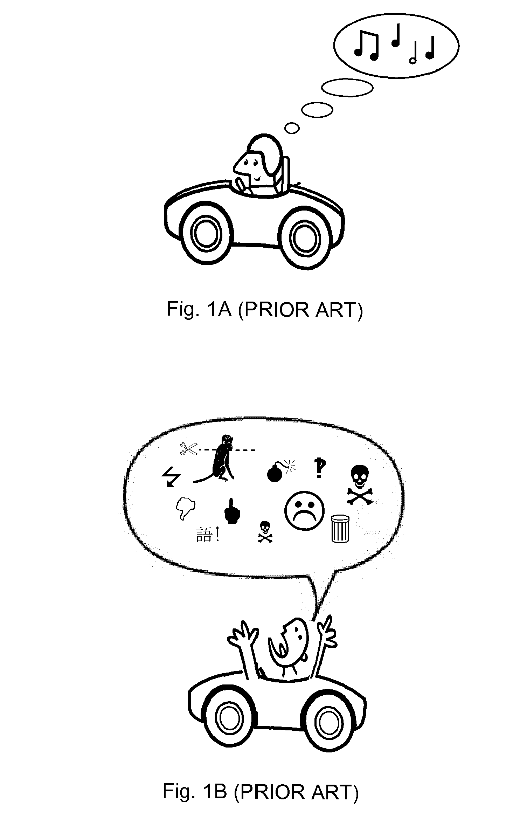

An automated or manual clutch control system like one of the above described, may also be regulated by a control algorithm, including parameters which may be impacted by the vehicles' driver, such as for example the position of the accelerator pedal, the brake pedal and/or the clutch pedal. Thus, the clutch multi-plate position may be chosen according to the control algorithm, based on driver input via for example any of the said pedal positions, position change or speed of the position change. For example, if the driver frequently depresses the accelerator pedal at the same time as the clutch pedal is released quickly, this may mean that the driver wishes to optimise against having a quick start and subsequently high vehicle acceleration. Additionally, certain automated clutch control systems are adapted to be regulated also by interpreting the speed at which the driver changes the accelerator pedal position and/or the clutch pedal position in various directions in order to estimate how the driver wishes the clutch system to behave and thus how the driver wants the vehicle to behave. This generally works well as long as the driver is in a harmonious state and/or acts rationally, see **Figure 1A**

One problem with the above described clutch control system is, however, the great dependency of appropriate behaviour on the part of the vehicle's driver. A driver in a traffic environment may be subject to and impacted by stressful traffic situations and therefore does not always behave rationally, see **Figure 1****B.** The driver may for example get stuck in a traffic jam at rush hour, while he or she has an appointment at a certain time; or be impacted emotionally by other drivers' perceived or actual flaws in the operation of their respective vehicles, which may trigger at least temporary irrational driver behaviour.

The automated clutch control system's control algorithms are adapted to a rational behaviour in the driver. Therefore, the clutch control system may behave irrationally if/when the driver behaves irrationally. For example, an extreme case of the above mentioned scenario where the driver depresses the accelerator pedal very quickly, while the clutch pedal is released quickly, may result in a tyre slippage, or so-called burn-out, where the vehicle's drive wheels skid on the ground, which in turn may lead to loss of side stability and in the worst case that the driver loses control over the vehicle, especially where the vehicle has a low weight combined with a powerful engine. In a heavily loaded vehicle, an overly rapid and insensitive disconnection combined with rapid acceleration may damage or break the drive shaft, or reduce its life.

It is likely that a driver who is afflicted by stress, irritation, anger and/or frustration (see figure 1B) may reinforce the signals which the clutch control system attempts to interpret, for example by quickly changing accelerator pedal and clutch pedal position from zero to full mode and back to zero again. Such flailing with the pedals may cause the clutch control system, due to problems of interpretation and potential time delay, to react contrary to what the driver actually wants, i.e. the clutch may give way when the driver actually wishes to drive forwards and/or vice versa. This may result in the vehicle loosing traction when the driver actually wants to increase traction in the stressful situation. Also, this behaviour may result in increased fuel consumption and thus increased environmental impact, but it may also result in increased wear and tear of the clutch, and/or the engine shaft, or an accident.

At the same time it is important that the clutch is sensitive to the driver's impact and that the driver is able to control the clutch with precision through a sensitive touch, such as in precision navigation at a loading dock or when reversing toward a trailer.

The driver, perhaps in particular professional drivers, are often subjected to situation-related stress. An irrationally acting driver is at risk of causing damage to the vehicle, in addition to the potential personal suffering which may follow.

### SUMMARY OF THE INVENTION

Therefore, one objective of this invention is to be able to adjust a control algorithm that controls a clutch control system in a vehicle, to a stress level in the vehicle's driver to resolve at least some of the above specified problems and thus achieve a vehicle improvement.

According to one first aspect of the invention, this objective is achieved through a method for the adjustment of a control algorithm comprising at least one driver dependent parameter, which control algorithm controls a clutch control system in a vehicle. The method comprises a measurement of at least one physical property of the driver. Further, the method comprises a determination of the driver's stress level, based on the measurement carried out. Further, the method comprises adaptation of the control algorithm to the driver's established stress level.

According to a second aspect of the invention, this objective is achieved by a control device for the control of a clutch control system in a vehicle via an adjustable control algorithm. The control device comprises a communications module to receive measurement results from a measurement of at least one physical property of the driver. The control device also comprises a processor circuit, arranged to establish the driver's stress level, based on the measurement carried out, and also arranged to adapt the control algorithm to the driver's established stress level.

According to a third aspect of the invention, this objective is achieved by a measuring device for provision of measurement results for the adjustment of a control algorithm, comprising at least one driver dependent parameter, whereby the control algorithm controls a clutch control system in a vehicle. The measuring device comprises a detector for the measurement of at least one physical property of the driver. Further, the measuring device comprises a communications module, arranged to communicate the measured value to a control device.

By measuring at least one physical stress-related parameter in the driver, and by being able to detect an increased stress level in the driver by comparing this stress-related parameter to a limit value, an increased stress level in the driver may be detected. The detected stress may then trigger an adjustment to the increased stress level in the driver of the control algorithm which controls the clutch control system in the driver's vehicle.. Thus, flailing driver inputs may be filtered out, attenuated and/or time delayed, with the objective of achieving a softer transmission. Thus, the wear and tear on the vehicle is reduced, in particular in relation to its clutch and power transmission, but also the risk of accidents to the vehicle and its driver is reduced. Thus, an improvement of the vehicle is achieved.

Other advantages and further, new features are set out in the detailed description of the invention, below.

### LIST OF FIGURES

The invention is described in further detail with reference to the enclosed figures, which illustrate embodiments of the invention:
- **Figure 1A**: is an illustration of a harmonious and rational vehicle driver.
- **Figure 1B**: is an illustration of a non-harmonious and irrational vehicle driver.
- **Figure 2**: is a summary illustration showing one embodiment of the invention.
- **Figure 3**: is a combined flow diagram and block diagram showing one embodiment of the invention.
- **Figure 4**: is a flow diagram illustrating one embodiment of a method for the adjustment of a control algorithm.
- **Figure 5**: is an illustration of a control device for the adjustment of a control algorithm according to one embodiment of the invention.
- **Figure 6**: is an illustration of a measuring device according to one embodiment of the invention.

### DETAILED DESCRIPTION OF THE INVENTION

The invention is defined as a method, a control device and a measuring device, which may be realised in any of the embodiments described below. This invention may, however, be carried out in many different forms and should not be seen as limited to the embodiments described herein, which are intended, instead, to illuminate and illustrate different aspects of the invention.

Further aspects and features of the invention may become apparent from the following detailed description when it is considered in combination with the enclosed figures. The figures should, however, only be seen as examples of various embodiments of the invention and should not be seen as limiting the invention, which is limited only by the enclosed claims. Further, the figures are not necessarily drawn to scale and are, unless otherwise indicated, intended to illustrate the aspects of the invention conceptually.

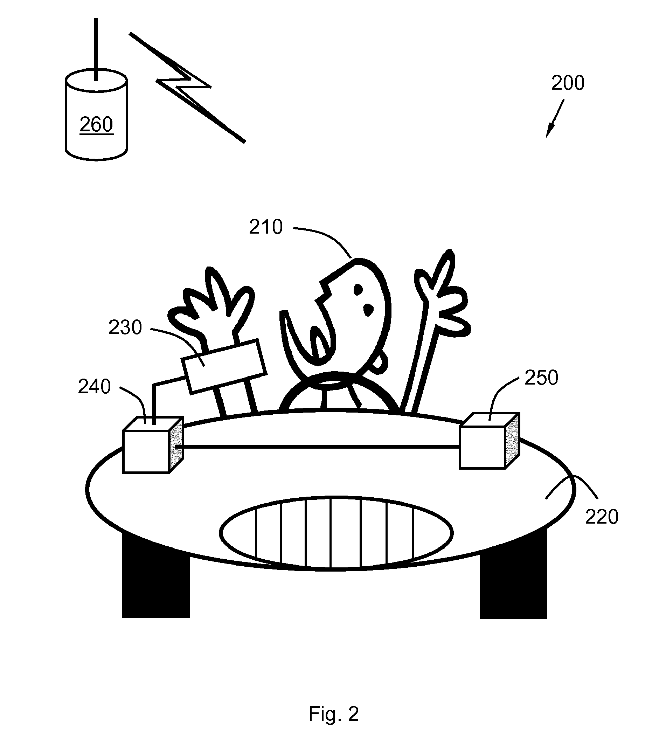

**Figure 2** shows a system **200** for the adjustment of a control algorithm. A driver **210** in front of a vehicle **220.** A measuring device **230** is adapted to read one or several physical parameters of the driver 210, for example blood pressure and/or heart rate. The measuring device 230 then sends these measurement values to a control device **240** through a wireless or wired connection. The control device 240 in turn impacts the vehicle's clutch control system **250.** An external storage device **260** is also adapted to receive and store measurement values which the measuring device 230 registers regarding the driver 210.

The measuring device 230 is adapted to measure one or several of the physical parameters in the driver 210, for example systolic blood pressure, diastolic blood pressure, heart rate, heart minus volume, vascular resistance, breathing volume, breathing frequency, body temperature, skin temperature, amount of adrenaline in the blood, amount of cortisol in the blood, amount of noradrenalin in the blood, transpiration volume, amount of hand sweat excreted, electrical conductivity of the skin, eye movement, muscle tension, body movement, speech volume, strength of the voice and/or size of pupils in the driver 210. Further, the system 200 may comprise several separate measuring devices 230 in certain embodiments.

In certain embodiments, the measuring device 230 is arranged to be worn by the driver 210 around the wrist. According to other embodiments, the measuring device 230 may be connected to another body part of the driver 210, such as a finger, hand, arm, and neck, part of the head, skin, torso, rectum, leg or foot. According to yet other embodiments, the measuring device 230 consists of several separate measuring devices, each of which measures different physical parameters of the driver 210 and sends these to the control device 240. The measuring device 230, or the measuring devices, may also in certain alternative embodiments be arranged to detect and measure physical parameters of the driver 210 at a distance and may be fitted e. g. in the driver's cabin and/or driver's seat. The concept of a measuring device 230 may thus also comprise several separate measuring devices located in the same or separate places on the driver 210, in the driver's vicinity, in the driver's cabin or at a certain distance from the driver 210 in various embodiments. One example is measuring device 230, which is adapted to measure the driver's skin temperature, and which may comprise a temperature sensor connected to the driver's skin in one embodiment, but alternatively may instead comprise an infrared camera adapted to measure the driver's skin temperature, located at a distance from the driver 210, for example in the driver's cabin.

Further, the measuring device 230 is arranged to communicate with a control device 240 via an interface, which may consist of a wireless interface in certain embodiments, but may also consist of a wired interface. The wireless interface may consist of a radio transmitter based on wireless communications technology such as 3rd Generation Partnership Project (3GPP) Long Term Evolution (LTE), LTE-Advanced, Evolved Universal Terrestrial Radio Access Network (E-UTRAN), Universal Mobile Telecommunications System (UMTS), Global System for Mobile Communications/ Enhanced Data rate for GSM Evolution (GSM/EDGE), Wideband Code Division Multiple Access (WCDMA), World-Wide Interoperability for Microwave Access (WiMax), Wireless Local Area Network (WLAN) Ultra Mobile Broadband (UMB), Bluetooth (BT) or an infra-red transmitter, mentioned herein as a few possible examples of wireless communication.

In certain embodiments, the measuring device 230 is designed to communicate one or several of the measured physical parameters in the driver 210 to the control device 240 via a communications bus. Such a communications bus is arranged to connect a number of electronic control devices (ECU's) or controllers, and different components arranged on the vehicle 220.

The control device 240 is in turn arranged to communicate with the measuring device 230, in order to receive measurement values and possibly also trigger a measurement, for example at a certain time interval. Further, the control device 240 is arranged to communicate with the vehicle's clutch control system 250, for example via the vehicle's communications bus, which may consist of one or several of: a cable, a bata bus, such as a CAN-bus (Controller Area Network bus), a MOST-bus (Media Oriented Systems Transport), or another bus configuration; or of a wireless connection for, example according to one of the technologies listed above.

The control device 240 may also in certain embodiments be integrated with the vehicle's clutch control system 250.

In certain embodiments the control device 240 is arranged to forward measurement values which the measuring device 230 registers in relation to the driver 210 to an external storage device 260, over a wireless interface, for example according to any of the previously listed technologies for wireless communication.

The external storage device 260 is also adapted to receive and store these measurement values, which may subsequently be analysed. Thus, a deteriorating health condition in individual drivers 210 may be detected and a suitable health promoting action package may be provided to the driver 210, with the objective of breaking the latter's negative health career, to prevent disease, sick leave and/or accidents. In certain embodiments, for example abuse of stimulating substances or performance impacting substances in the driver 210 may be detected. The stored measurement values may also be used, for example, to investigate accidents which have occurred, and may facilitate the detection of physical parameter(s) or change(s) of parameter(s) preceding an accident. Such detection may be used to further improve the control algorithm for the control system 250, and also other control algorithms in the vehicle, applied to for example the vehicle's brake system or similar.

The vehicle's clutch control system 250 is arranged to impact and control the clutch in a gearbox. The gearbox may be a manual gearbox, an automatic gearbox or an automated manual gearbox in different embodiments. Also, the clutch may consist of an automated clutch which may for example be of dry plate type. The friction element's (plate's) engagement with the flywheel on the engine output shaft may be controlled with the help of a pressure plate which is displaceable laterally by means of e.g. a lever, whose function is controlled by a clutch actuator. The clutch actuator's impact on the lever is in turn controlled by the vehicle's clutch control system 250.

The control algorithm which controls the vehicle's clutch control system 250 is impacted by one or more parameters, which may be driver dependent, indirectly driver dependent or driver independent, such as for example the vehicle's inclination, vehicle weight, vehicle type, ride comfort, performance choices, vehicle speed and/or engine speed.

Through the embodiments described herein, aggressive or irrational behaviour on the part of the driver 210 may be detected and its consequences to the clutch control system 250 and the vehicle 220 may be reduced. However, a health condition which is dangerous in vehicular traffic such as blood circulation disruptions, fall in blood pressure, fainting, heart attack, stroke, epileptic seizures or similar may also be detected and/or predicted and its consequences to the clutch control system 250 and the vehicle 220 may be reduced.

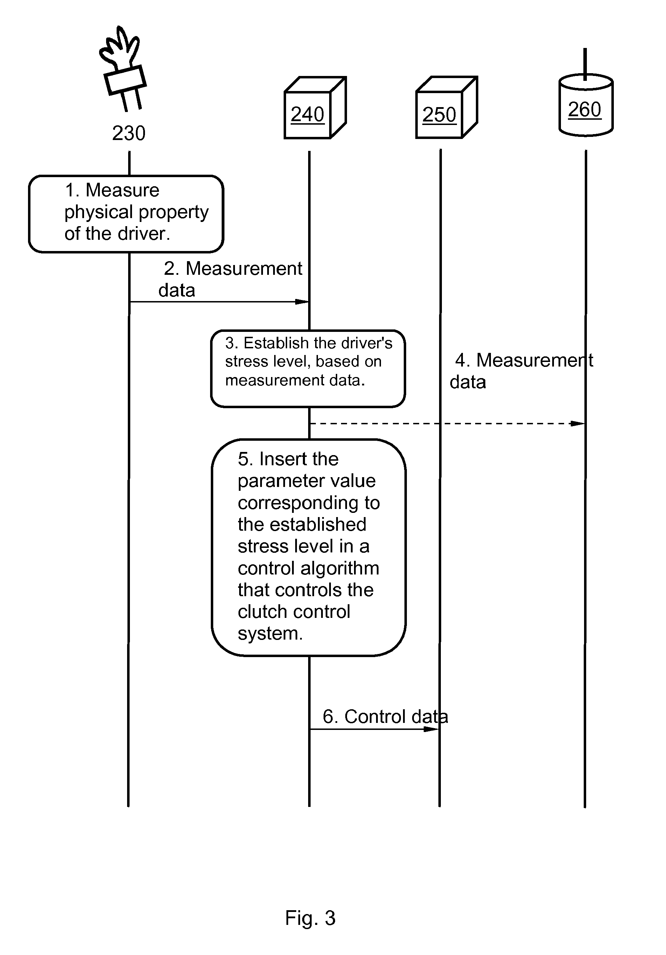

**Figure 3** illustrates a summary example of the invention, divided into a number of steps 1-6. Some of these steps are not necessarily included in all embodiments of the invention. Further, these steps may in some embodiments be carried out in a different sequence than as indicated by the numbering. Certain steps may potentially also be carried out in parallel with each other.

### Step 1

The measuring device 230 measures a physical property of the driver 210. This physical property may consist of e. g. blood pressure or heart rate.

### Step 2

This measured physical property is sent to the control device 240 via wireless or wired signalling, for example via the vehicle's communications bus.

### Step 3

The control device 240 receives measurement values for at least one physical property of the driver 210. These measurement values are used to establish the driver's stress level by for example comparing the received measuring value to a limit value, by detecting the size of a difference between two measured values, and/or by detecting the speed of a change between two measured values.

### Step 4

In certain embodiments, the received measurement values are forwarded from the control device 240 to an external data storage device 260, in order to facilitate for example a health analysis of the driver 210.

### Step 5

The control device 240 is arranged to adapt a control algorithm comprising at least one driver dependent parameter, which control algorithm controls the clutch control system 250 in the vehicle 220 to the driver's established stress level.

This adaptation of the control algorithm may in certain embodiments comprise a filtering away of the clutch pedal movements, or another driver dependent parameter, and/or a time filtering of the driver's clutch request, with the objective of achieving a softer transmission.

In certain embodiments, the control algorithm is adapted to the driver's established stress level by attenuating a driver dependent parameter, proportionally to the driver's stress level.

One advantage which is thus achieved is that the vehicle 220, or rather its clutch, will be subjected to a more even wear and tear regardless of which driver 210 who drives the vehicle 220 and regardless of the mental state of such driver 210 (calm/upset). Thus, stress-level related differences in driving styles of different drivers 210 are neutralised, which results in less wear and tear but also greater predictability of the vehicle's behaviour in relation to other drivers, which in turn may have an effect in reducing accidents.

### Step 6

Control data, corrected and adjusted to the driver's stress level is then sent to the clutch control system 250 in order to impact and control the clutch manoeuvring via the latter.

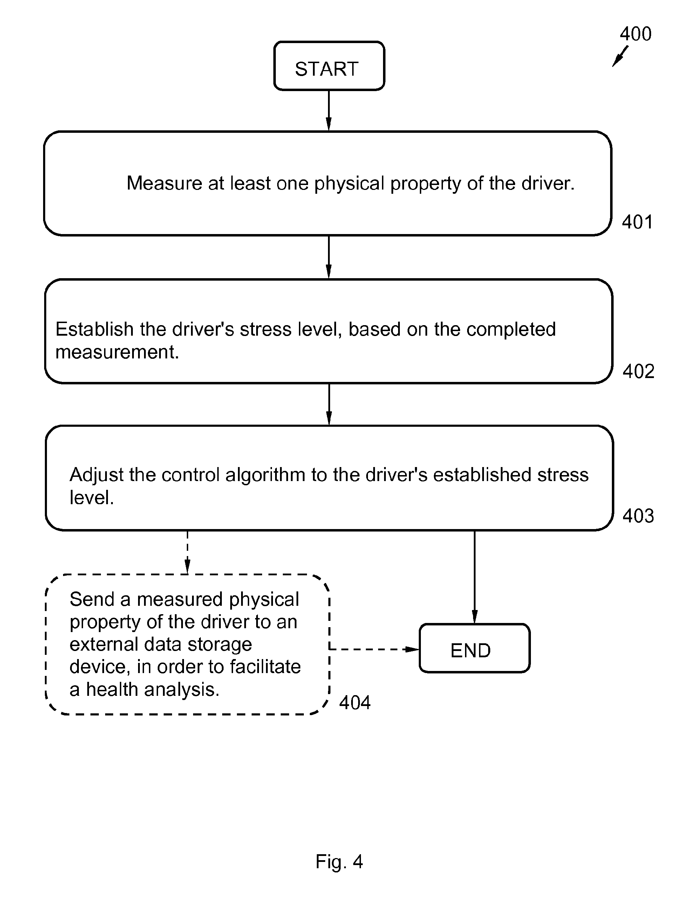

**Figure 4** illustrates an example of an embodiment of the invention. A flow diagram in figure 4 illustrates a method **400** for the adjustment of a control algorithm comprising at least one driver dependent parameter, which control algorithm controls a clutch control system 250 in a vehicle 220.

The control algorithm may in some embodiments comprise at least one driver dependent parameter of: position of a accelerator pedal, position of a clutch pedal, position of a brake pedal, position change of a accelerator pedal, position change of a clutch pedal, position change of a brake pedal, position change per time unit of a accelerator pedal, position change per time unit of a clutch pedal, position change per time unit of a brake pedal, steering angle and/or speed of steering angle change. The term "position of gas-/clutch-/brake pedal" means the extent to which the pedal has been depressed by the driver 210; and not the location of the pedal in the room, i.e. the driver's cabin.

Further, the control algorithm may in some embodiments comprise at least one driver independent parameter of: vehicle type, inclination of the vehicle 220, vehicle weight, comfort level. In certain configurations, the parameter comfort level may be adjustable by either the driver 210, the vehicle's owner, the vehicle manufacturer or another party.

The objective of the method is to adjust the control algorithm to the driver's heightened stress level, whereby the parameters which are not directly driver dependent obtain a proportionally higher weighting in relation to the directly driver dependent parameters.

In order to be able to adjust the control algorithm correctly, the method 400 may comprise a number of steps **401-404.** It should be noted, however, that some of the steps described herein are only included in certain alternative embodiments of the invention, such as e. g. step 404. Further, the steps described herein may be carried out in a somewhat different chronological order than suggested by the numbering, and some of these may be completed in parallel with each other. The method 400 comprises the following steps:

### Step 401

A measurement of at least one physical property of the driver 210 is carried out. This measurement may be carried out by one or several measuring devices 230.

The measurement of the physical property of the driver 210 may be carried out by the measuring device 230, which may for example be designed as a cuff, and possible to connect to the driver's arm or another body part of the driver 210 in certain embodiments, for example a finger, hand, arm, neck, part of the head, skin, torso, rectum, leg or foot. According to yet other embodiments, the measuring device 230 consists of several separate measuring devices, each of which measures different physical parameters of the driver 210 and sends these to the control device 240. The measuring device 230, or the measuring devices, may also in certain alternative embodiments be arranged to detect and measure physical parameters of the driver 210 at a distance and may be fitted e. g. in the driver's cabin and/or driver's seat. The concept of a measuring device 230 may thus also comprise several separate measuring devices located in the same or separate places on the driver 210, in the driver's vicinity, in the driver's cabin or at a certain distance from the driver 210 in various embodiments. The measured physical property of the driver 210 may be sent via wireless or wired cable connection to a control device 240.

However, the measuring device 230 may comprise a number of measuring devices, or a measuring device 230 comprising a number of different detectors, which may have the same or different spatial locations.

In one embodiment, the measuring device 230 may comprise, or at least partly comprise the vehicle's horn, since the emotionally exalted driver 210 likely has a tendency to overuse the horn.

Further, the measured physical property of the driver 210 may comprise at least one of: systolic blood pressure, diastolic blood pressure, heart rate, heart minus volume, vascular resistance, breathing volume, breathing frequency, body temperature, skin temperature, amount of adrenaline in the blood, amount of cortisol in the blood, amount of noradrenalin in the blood, transpiration volume, amount of hand sweat excreted, electrical conductivity of the skin, eye movement, muscle tension, body movement, speech volume, strength of the voice and/or size of pupils in the driver 210, according to different embodiments.

The measured 401 physical property of the driver 210 may in some embodiments comprise a level change in at least one measured physical property of the driver 210.

In some embodiments, the measured physical property of the driver 210 comprises a level change per time unit in at least one measured physical property of the driver 210.

In some forms, only one single physical driver property is measured, which leads to a quick and simple analysis, and a reduced price tag of the measuring device 230. In addition, only a limited amount of data needs to be transmitted to the control device 240, and also processed and analysed in this device.

One advantage of measuring several physical properties of the driver 210 is that the reliability and/or sensitivity of the measurement increases. Changes in one single of the above mentioned parameters as such may have an entirely different, non-stress related cause. For example, an increased skin temperature of the driver 210 may be due to a jam in the heating system in the driver's cabin, rather than the driver 210 being on the verge of an affective outburst of anger or bitterness. In the same manner, for example dilated pupils in the driver 210 may be due to a meeting vehicle not having dimmed its headlights or having misaligned headlights. By measuring a number of parameters, such as for example the above mentioned ones, at least some potential alternative explanation models of the driver's discrepant physical measurement values may be filtered away, which increases the accuracy of this method according to these embodiments.

The measurement of the driver's physical property or properties may be carried out continuously, or at certain time intervals according to some embodiments. The measurement may also be triggered by another party or event, such as vehicle start, vehicle stop, or similar.

In certain embodiments, an immobilizer of the vehicle 220 is activated when the driver 210 removes the measuring device 230, which is not cancelled until the driver 210 returns the measuring device 230 to its place.

In certain alternative embodiments, an immobilizer of the vehicle 220 is activated when the measuring device 230 detects physical parameters which differ from the normal parameters of the ordinary driver by a certain threshold. Thus, it is possible to prevent that another individual, such as an unauthorised driver or a thief may start and/or drive the vehicle 220.

In some other alternative embodiments, an immobilizer is activated in the vehicle 220 when the measuring device 230 detects physical parameters that differ from the driver's ordinary normal parameters by a certain threshold level. Thus, it is possible to avoid that an emotionally affected driver 210 begins to drive the vehicle.

### Step 402

The driver's stress level is established, based on the measurement carried out.

This establishment of the driver's stress level may be based on the measurement carried out, and comprise a comparison between the measured value and a limit value.

For example, the physical parameter measured may consist of the driver's heart rate. The driver's measured heart rate, which may be for example 72 beats per minute, may then be compared to a limit value which may be predetermined, or configurable to for example 85. The limit value may also be adjusted to each individual driver 210, and for example be at 10% above the driver's heart rate at rest. When the driver's heart rate in this example falls below the limit value, the driver 210 may be categorised as harmonious, or unstressed. In such cases, the ordinary control algorithm may be used to impact the clutch control system, intact, without adjustment to the driver's measurement values since the driver 210 may be deemed to be harmonious. However, if the driver's heart rate on a different occasion exceeds 85 beats per minute, the driver 210 may be categorised as stressed, which in turn may trigger an adjustment of the control algorithm according to the following steps.

One advantage is thus that the control algorithm is not impacted at all when the driver 210 is unstressed. Thus, the clutch system's sensitivity may be maintained for the unstressed driver 210, while an attenuation of the driver dependent parameters may be inserted in the clutch control system 250 for a stressed driver, which results in an improved clutch control system 250.

The physical parameter measured in other examples may consist of the driver's changed heart rate. Assuming that the driver's heart rate is 72 beats per minute on one first measuring occasion and 85 beats per minute one a second measuring occasion; the increase in heart rate, then, is 85-72 = 13 beats per minute, which value may be compared to a limit value, which may be for example 7 beats per minute, or for example 10% of the original value (i.e. of 72). One advantage with measuring changes in a driver's physical parameters is that the same limit value may be used regardless of which individual driver 210 is measured. Thus, it is not necessary to calibrate the limit value for each individual driver 210 of the vehicle 220.

Also, according to certain further embodiments, the physical parameter measured in other additional examples may consist of the driver's heart rate changes per time unit. Assuming that the driver's heart rate is 72 beats per minute on one first measuring occasion, and 85 beats per minute one a second measuring occasion, which is one minute later; the increase in heart rate is 85-72 = 13 beats per minute, which value may be compared to a limit value, which may be for example 7 beats per minute, or for example 10% of the original value (i.e. of 72). This embodiment also has the advantage that the same limit value may be used regardless of which individual driver 210 who is measured.

### Step 403

The control algorithm is adjusted to the driver's established stress level 402.

This adjustment of the control algorithm comprises, according to some embodiments, a reduction of the driver dependent parameter value which is proportionate to the driver's established stress level 402.

For example, a measuring value of a driver dependent parameter which is below a limit value may reduce the driver dependent parameter value by 0%, a measuring value of a driver parameter which is equal to the limit value may reduce the driver dependent parameter value by 10%, while a measuring value of a driver parameter that exceeds the limit value by 10% may reduce the driver dependent parameter value by 30%, herein mentioned herein as a random and non-limiting example.

The adjustment of the control algorithm may in certain embodiments comprise a mapping of the driver's established stress level 402 against a correspondingly reduced parameter value and the introduction of this reduced parameter value into the control algorithm.

Thus, it is possible to compare the measurement values of the measured driver parameter with a measurement value or measurement value interval in a table, and through this mapping to extract a correspondingly reduced parameter value, which may then be introduced into the control algorithm to attenuate the affected driver's clutch requests and thus to ensure a softer transmission.

Also, the adjustment of the control algorithm may comprise the introduction of a time filter, where the length of the time delay is proportionate to the driver's established stress level 402.

For example, a measuring value of a driver parameter which is below a limit value may cause zero time delay; a measuring value of a driver parameter equal to the limit value may cause a time delay of one second, while a measuring value of a driver parameter which exceeds the limit value by 10% may cause a time delay of three seconds, herein mentioned as a random and non-limiting example.

### Step 404

This method step is only carried out in certain alternative embodiments of the method 400.

According to these alternative embodiments, the measured physical property 401 of the driver 210 is sent to an external data storage device 260, to facilitate a health analysis of the driver 210.

The external data storage device 260 may be located for example at the owner of the vehicle or a similar location, and be arranged to store physical properties of the driver 210, or a number of drivers, together with information regarding for example time specifications and/or geographical position. In addition, the external storage device 260 may be arranged to analyse these stored measurement values. This information may then be used to detect a deteriorated health condition of the individual driver 210 and professional help may then be summoned to prescribe further measures in order to stop the negative development of the sick driver's health.

The stored measurement values may also be used for example to investigate accidents which have occurred, in a manner similar to the "black box" in an aircraft. Thus, the stored measurement values with the driver's detected emotional eruptions may be analysed, facilitating for example the detection of the physical parameter(s) or parameter change(s) which precede an accident. Such detection may be used to further improve the control algorithm for the control system 250, and also other control algorithms, e. g. for the vehicle's brake system or similar.

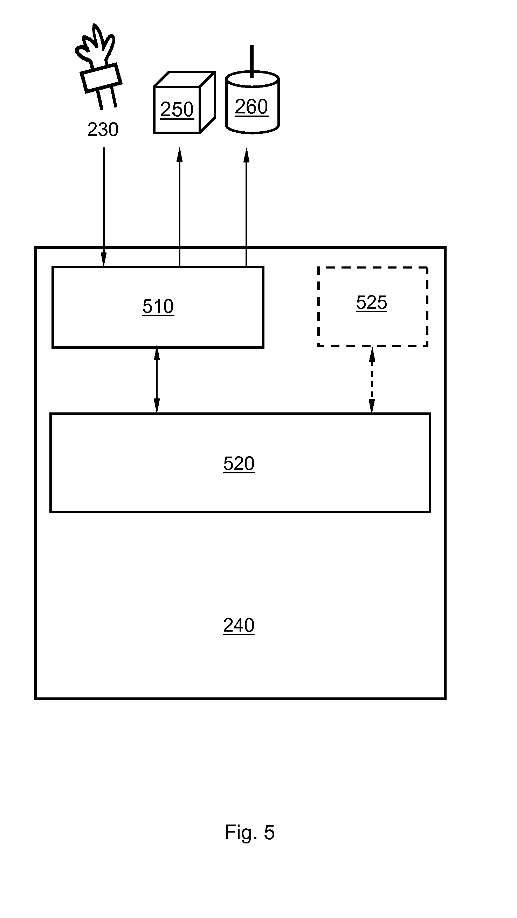

**Figure 5** illustrates one embodiment of a control device 240 for the control of a clutch control system 250 in a vehicle 220 via an adjustable control algorithm. This control device 240 is configured to complete at least some of the previously described steps 401-404, included in the description of the method 400 for the adjustment of a control algorithm, comprising at least one driver dependent parameter regarding a driver's stress level, which control algorithm controls a clutch control system 250 in a vehicle 220.

The driver's stress level is established based on a measured physical property of the driver 210 comprising at least one of: systolic blood pressure, diastolic blood pressure, heart rate, heart minus volume, vascular resistance, breathing volume, breathing frequency, body temperature, skin temperature, amount of adrenaline in the blood, amount of cortisol in the blood, amount of noradrenalin in the blood, transpiration volume, amount of hand sweat excreted, electrical conductivity of the skin, eye movement, muscle tension, body movement, speech volume, strength of the voice and/or size of pupils, according to different embodiments.

The measured physical property of the driver 210 may comprise for example a level change of a least one measured physical property of the driver 210 in certain embodiments. Further, the measured physical property of the driver 210 may comprise a level change per time unit in at least one measured physical property of the driver 210.

In order to correctly adjust the control algorithm to the driver's stress level, the control device 240 includes a number of components, which are described in more detail in the text below. Some of the partial components described only occur in certain, but not necessarily all, embodiments. There may also occur additional electronic components in the control device 240, which are not entirely necessary in order to understand the function of the control device 240 according to the invention.

The control device 240 comprises a communications module 510 for receiving a measuring result from a measurement of at least one physical property of the driver 210. The measurement result is received through a wireless or wired interface from a measuring device 230. Also, the communications module 510 is arranged to send instructions to a clutch control system 250, which in turn impacts the vehicle's clutch and clutch response. The communications module 510 is also in some embodiments arranged to send measurement data to an external device 260, for storage and analysis of such measurement data. This communication is wireless to the external storage device 260.

The communications module 510 may in certain embodiments consist of a separate sender and receiver. The communications module 510 may in some embodiments consist of a transceiver, which is adapted to send and receive radio signals, and where parts of the construction, such as the antenna, are joint to sender and receiver. Further, the communications module 510 may be adjusted to wireless information transfer, via radio waves, WLAN, Bluetooth or an infrared sender/ receiver module. However, the communications module 510 may in certain embodiments be especially adapted to wired information exchange with the measuring device 230 and/or the vehicle's bata bus.

The control device 240 also comprises a processor circuit 520, arranged to establish the driver's stress level, based on the measurement carried out, and also arranged to adapt the control algorithm to the driver's established stress level.

In certain embodiments, the processor circuit 520 may be arranged to establish the driver's stress level based on a comparison between the measured value and a limit value.

In certain embodiments, the processor circuit 520 may be arranged to adjust the control algorithm by reducing the driver dependent parameter's value proportionately to the driver's established stress level.

For example, a measuring value of a driver dependent parameter which is below a limit value may reduce the driver dependent parameter value by 0%, a measuring value of a driver parameter which is equal to the limit value may reduce the driver dependent parameter value by 10%, while a measuring value of a driver parameter that exceeds the limit value by 10% may reduce the driver dependent parameter value by 30%, herein mentioned herein as a random and non-limiting example.

In some embodiments, the processor circuit 520 may be arranged to adjust the control algorithm by mapping the driver's established stress level against a correspondingly reduced parameter value and introducing this reduced parameter value into the control algorithm.

Thus, it is possible to compare the measurement values of the measured driver's parameter with a measurement value or measurement value interval in a table, and through this mapping to extract a correspondingly reduced parameter value, which may then be introduced into the control algorithm to attenuate the affected driver's clutch requests and thus to ensure a softer transmission.

In certain embodiments, the processor circuit 520 may be arranged to adjust the control algorithm by introducing a time filter, where the time delay is proportionate to the driver's established stress level.

For example, a measuring value of a driver parameter which is below a limit value may give rise to a zero time delay; a measuring value of a driver parameter which is equal to the limit value may cause a time delay of one second, while a measuring value of a driver parameter which exceeds the limit value by 10% may cause a time delay of three seconds, herein mentioned as a random and non-limiting example.

The processor circuit 520 may consist of for example one or several Central Processing Units (CPU), a microprocessor or other logic designed to interpret and execute instructions and/or such as to read and write data. The processor circuit 520 may handle data for inflow, outflow or data processing of data comprising also buffering of data, control functions and similar.

In certain alternative embodiments, the control device 240 comprises a memory device 525, consisting of a storage medium for data. The memory device 525 may for example consist of a memory card, flash memory, USB-memory, hard disk or other similar data storage device, for example one from the group: ROM (Read-Only Memory), PROM (Programmable Read-Only Memory), EPROM (Erasable PROM), Flash-memory, EEPROM (Electrically Erasable PROM), etc. in various embodiments.

Further, the invention comprises a computer program for the adjustment of a control algorithm comprising at least one driver dependent parameter, whereby the control algorithm controls a clutch control system 250 in a vehicle 220. The computer program is arranged to execute the method 400 according to at least one of the previously described steps 401-404, when the computer program is executed in a processor circuit 520 in the control device 240.

The method according to the steps 401-404 for the adjustment of a control algorithm to a driver's stress level may be implemented by one or several processor circuits 520 in the control device 240, together with computer program code to execute one, several, certain or all of the steps 401-404 described above. Thus, a computer program comprising instructions to execute the steps 401-404 may then be loaded in the processor circuit 520.

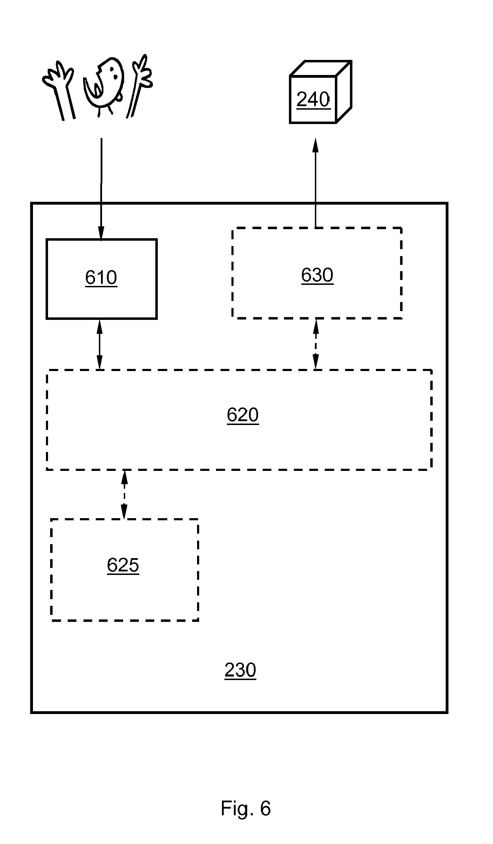

**Figure 6** shows a measuring device 230. The measuring device 230 is arranged to provide measuring results for the adjustment of a control algorithm comprising at least one driver dependent parameter, whereby the control algorithm controls a clutch control system 250 in a vehicle 220.

The measuring device 230 may comprise or at least partly consist of a blood pressure monitor, heart rate monitor, a humidity sensor, a thermometer, a camera, an infrared camera, a movement detector, a microphone, a voice analysis device, a horn, a mobile telephone, portable computer, towed PC, tablet, netbook, PDA or similar.

In order to provide correct measurement results of at least one driver dependent physical parameter, the measuring device 230 contains a number of components, which are described in further detail below. Some of the partial components described only occur in certain embodiments. Further, additional electronic components in the measuring device 230, which are not strictly necessary in order to understand the function of the measuring device 230 according to the invention, have not been depicted in figure 6, to avoid unnecessarily complicating the understanding of the invention.

The measuring device 230 comprises a detector 610 for the measurement of at least one physical property of the driver 210.

The detector 610 is further arranged to measure at least one of the following physical properties in the driver 210: systolic blood pressure, diastolic blood pressure, heart rate, heart minus volume, vascular resistance, breathing volume, breathing frequency, body temperature, skin temperature, amount of adrenaline in the blood, amount of cortisol in the blood, amount of noradrenalin in the blood, transpiration volume, amount of hand sweat excreted, electrical conductivity of the skin, eye movement, muscle tension, body movement, speech volume, strength of the voice and/or size of pupils.

The measuring device 230 may in certain embodiments comprise a number of detectors 610, which are adapted to measure different physical properties in the driver 210, from among the properties listed above. These detectors 610 may be located in a number of places in the driver's cabin and/or on the driver 210. Examples may be the steering wheel of the vehicle which may comprise a humidity sensor, thermometer and/or measuring element for electrical conductivity.

One advantage of measuring several physical properties of the driver 210 is that the reliability and/or sensitivity of the measurement increases. Changes in one single of the above mentioned parameters as such may have an entirely different, non-stress related cause. For example, an increased skin temperature of the driver 210 may be due to a jam in the heating system in the driver's cabin, rather than the driver 210 being on the verge of an affective outburst of anger. In the same manner, for example dilated pupils in the driver 210 may be due to a meeting vehicle not having dimmed its headlights or having misaligned headlights, rather than to the driver's anger.

The measuring device 230 also comprises a communications module 630 arranged to communicate the measured value to a control device 240. The communications module 630 is in turn arranged to communicate the measured value wirelessly or via a cable to the control device 240 and/or a data storage device 260, in certain embodiments.

The communications module 630 may in some embodiments consist of a separate sender and receiver. The communications module 630 may in some embodiments consist of a transceiver, which is adjusted to send and receive radio signals, and where parts of the construction, for example the antenna, are joint to the sender and receiver. Further, the communications module 630 may be adapted to wireless information transfer, via radio waves, WLAN, Bluetooth or an infrared sender/ receiver module. However, the communications module 630 may in some embodiments be especially adapted to wired information exchange with the control device 240 and/or the vehicle's bata bus.

In some embodiments the measuring device 230 comprises a camera, adapted to measure the driver's pupil size. In at least some of these embodiments, the measuring device 230/the camera can be fitted in a location in front of the driver 210 in the driver's cabin, such as for example in the instrument panel, the steering wheel, the rear view mirror, the driver's cabin's ceiling or in a similarly suitable place essentially in front of the driver 210, so that the driver's pupil may be detected and the pupil size may be measured.

In some embodiments, the measuring device 230 is designed as a cuff, which may be connected to the driver's arm or another body part. Thus, for example the driver's blood pressure, heart rate, skin temperature, electrical conductivity of the skin and/or amount of transpiration may be measured easily and reliably.

In one embodiment, the vehicle 220 may not be started unless the driver 210 applies the measuring device 230.

The measuring device 230 is in some embodiments arranged to detect a level change in at least one measured physical property of the driver 210. Alternatively, the measuring device 230 may be adapted to detect a level change per time unit in at least one measured physical property of the driver 210.

Further, some embodiments of the invention comprise a system 200 for the adjustment of a control algorithm comprising at least one driver dependent parameter, which control algorithm controls a clutch control system 250 in a vehicle 220. This system 200 comprises a measuring device 230, according to one of the embodiments described in connection with the presentation of figure 6. System 200 also comprises a control device 240, according to any of the embodiments described in connection with the presentation of figure 5, which is adapted to establish the stress level of the driver 210, based on the measurement made and to adjust a control algorithm to the driver's established stress level.

Some embodiments of the invention also comprise a vehicle 220, which comprises the above described system 200.

According to some alternative embodiments the vehicle 220 also comprises, or may be connected to, a device to establish the geographical position, such as a GPS module. This alternative device is specifically adapted to establish the vehicle's existing position, and facilitates for example a storage or marking to mark the position for a stress incident in the driver 210, potentially jointly with time information. Thus, the geographical position and/or point in time for stress reactions in the driver 210 and/or a number of drivers may be sent to and stored in the external data storage device 260 for later presentation. For example, a stress map may then be drawn, based on this gathered information, which may be used to plan a route, or to warn the driver 210 that he or she is travelling in a high stress area, based on the latter's geographical position/position change direction. Thus, harmful stress situations may be eliminated or their consequences may at least be reduced, by the route being planned so that high stress areas/times are avoided.

Further, according to certain alternative embodiments, where a heightened stress level is detected in the driver 210, feedback may be provided to the driver 210, in order to calm the driver. Such feedback may for example consist of calming/ relaxing music/noise/speech, or increased ventilation/air conditioning in the driver cabin with the objective of reducing the temperature and increasing oxygenation, or a massage module built into the driver's seat may be activated, which may have a pacifying impact on the mentally upset driver 210. Thus, the risk of stress-related injuries and/or accidents may be further reduced.

It may also be concluded that embodiments of the invention are applicable both to gear changes for travelling forwards and for gear changes for backing of the vehicle 220, respectively. However, on some occasions, it may be advantageous only to activate the above specified method when travelling in the vehicles' forward direction, in order to reduce the risk of triggering further driver aggression in connection with for example backing or parallel parking. Further, certain embodiments may comprise selectively shutting off function according to the invention in connection with certain manoeuvres, such as driving the vehicle 220 in a shunting /manoeuvring mode where the driver 210 often drives the vehicle 220 at a very low speed when for example calling at a loading dock.

Additionally, the invention above has, according to some embodiments, been exemplified for a situation where the driver 210 requests momentum via a driver controlled acceleration element such as a accelerator pedal. However, the invention is applicable also where the driver 210 requests momentum in any other manner, such as via some driver assisted system or other applicable control device.

The present invention may be used both for pedal driving, i.e. when the driver 210 regulates the torque demand from the engine, and for cruise control driving. The term pedal driving comprises, here and in this document, essentially all kinds of controls designed to control torque request, such as for example a accelerator pedal or a hand gas device.

## Claims

1. Method (400) for the adjustment of a control algorithm comprising at least one driver dependent parameter, which control algorithm controls a clutch control system (250) in a vehicle (220), the method comprising:
measurement (401) of at least one physical property of the driver (210); **characterised by**
establishment (402) of the driver's stress level, based on the completed measurement (401); and
adjustment (403) of the control algorithm to the driver's established (402) stress level, whereby the adjustment (403) of the control algorithm comprises a reduction of the driver dependent parameter value which is proportional to the driver's established (402) stress level.

2. Method (400) according to claim 1, wherein the measured physical property of the driver (210) comprises at least one of: systolic blood pressure, diastolic blood pressure, heart rate, heart minus volume, vascular resistance, breathing volume, breathing frequency, body temperature, skin temperature, amount of adrenaline in the blood, amount of cortisol in the blood, amount of noradrenalin in the blood, transpiration volume, amount of hand sweat excreted, electrical conductivity of the skin, eye movement, muscle tension, body movement, speech volume, strength of the voice and/or size of pupils.

3. Method (400) according to claim 1 or claim 2, wherein the measured (401) physical property of the driver (210) comprises a level change, or a level change per time unit, in at least one measured physical property of the driver (210) .

4. Method (400) according to claims 1-3, wherein the establishment (402) of the driver's stress level, based on the measurement carried out (401), comprises a comparison between the measured value and a limit value.

5. Method (400) according to claims 1-4, wherein the adjustment (403) of the control algorithm comprises a mapping of the driver's established (402) stress level against a correspondingly reduced parameter value and the introduction of this reduced parameter value into the control algorithm.

6. Method (400) according to claims 1-5, wherein the adjustment (403) of the control algorithm comprises the introduction of a time filter, the length of the time delay being proportionate to the driver's established (402) stress level.

7. Method (400) according to claims 1-6, wherein the control algorithm comprises at least one of:
a driver dependent parameter of: position of a accelerator pedal, position of a clutch pedal, position of a brake pedal, change in position of accelerator pedal, change in position of clutch pedal, change of position of brake pedal, change of position per time unit of accelerator pedal, change of position per time unit of clutch pedal, change of position per time unit of brake pedal, steering angle and/or speed of steering angle change; and/or
one driver independent parameter of: vehicle type, inclination of the vehicle (220), vehicle weight, comfort level.

8. Method (400) according to claims 1-7, wherein measurement (401) of at least one physical property of the driver (210) is carried out by a measuring device (230), which may be connected to a body part of the driver 210, the measured (401) physical property of the driver (210) being sent wirelessly or via cable to a control device (240).

9. Method (400) according to claims 1-8, comprising:
sending (404) of measured (401) physical property of the driver (210) to an external data storage device (260) to facilitate a health analysis of the driver (210).

10. Computer program for the adjustment of a control algorithm comprising at least one driver dependent parameter, the control algorithm controlling a clutch control system (250) in a vehicle (220), which computer program **is characterised by** carrying out the method (400) according to any of the claims 1-9, when the computer program is executed in a processor circuit (520) in a control device (240).

11. Control device (240) for the control of a clutch control system (250) in a vehicle (220) via an adjustable control algorithm, the control device (240) comprising:
a communications module (510) for receiving measuring results from a measurement of at least one physical property of the driver (210); **characterised by**
a processor circuit (520) arranged to establish the driver's stress level, based on the measurement carried out, and also arranged to adjust the control algorithm to the driver's established stress level, the processor circuit (520) being arranged to adjust the control algorithm by reducing the driver dependent parameter value proportionately to the driver's established stress level.

12. Control device (240) according to claim 11, in wherein the measured physical property of the driver (210) comprises at least one of: systolic blood pressure, diastolic blood pressure, heart rate, heart minus volume, vascular resistance, breathing volume, breathing frequency, body temperature, skin temperature, amount of adrenaline in the blood, amount of cortisol in the blood, amount of noradrenalin in the blood, transpiration volume, amount of hand sweat excreted, electrical conductivity of the skin, eye movement, muscle tension, body movement, speech volume, strength of the voice and/or size of pupils.

13. Control device (240) according to either of claims 11 or 12, wherein the measured physical property of the driver (210) comprises a level change or a level change per time unit in at least one measured physical property of the driver (210).

14. Control device (240) according to any of the claims 11-13, wherein the processor circuit (520) is arranged to establish the driver's stress level based on a comparison between the measured value and a limit value.

15. Control device (240) according to any of the claims 11-14, wherein the processor circuit (520) is arranged to adjust the control algorithm by mapping the driver's established stress level against a correspondingly reduced parameter value and introducing this reduced parameter value into the control algorithm.

16. Control device (240) according to any of the claims 11-15, wherein the processor circuit (520) is arranged to adjust the control algorithm by introducing a time filter, the length of the time delay being proportionate to the driver's established stress level.

17. System (200) for the adjustment of a control algorithm comprising at least one driver dependent parameter, which control algorithm controls a clutch control system (250) in a vehicle (220), the system comprising
a measuring device (230), adjusted to measure at least one physical property in the driver (210); **characterised by**
a control device (240) according to any of the claims 11-16, adjusted to establish the driver's (210) stress level, based on the measurement carried out and to adjust the control algorithm to the driver's established stress level by reducing the driver dependent parameter value proportionately to the driver's established stress level.

18. Vehicle (220) **characterised by** comprising a system (200) according to claim 17.
